# EUROPEAN PATENT APPLICATION

(11) **EP 2 130 521 A1**
(43) Date of publication of application: **09.12.2009**
(21) Application number: 08722650.2
(22) Date of filing: 21.03.2008
(51) Int. Cl.: A61F 13/15, A61F 13/53, A61F 13/534

(54) **ABSORBENT ARTICLE AND METHOD OF PRODUCING THE ABSORBENT ARTICLE**

(30) Priority: 26.03.2007 JP 2007079926; 26.03.2007 JP 2007079928
(71) Applicant: Uni-Charm Corporation, Ehime 7990111 (JP)
(72) Inventor: NODA, Yuki, Kanonji-shi Kagawa 769-1602 (JP); KURODA, Kenichiro, Kanonji-shi Kagawa 769-1602 (JP); NISHIKAWA, Kumiko, Kanonji-shi Kagawa 769-1602 (JP)
(74) Representative: Sperling, Rüdiger
(86) International application number: PCT/JP2008/055298
(87) International publication number: WO 2008/117754

(57) **Abstract**

Bulging that occurs due to swelling of superabsorbent resin in an absorbent article is suppressed. The present invention includes an absorbent article including an absorbent body material including an accumulated absorbent fiber and a superabsorbent resin, wherein the absorbent body material includes interspersed interspersed sections that are interspersed, and a densely gathered state of the absorbent fiber in the interspersed sections is less dense than a densely gathered state of the absorbent fiber in areas surrounding the interspersed sections, and an occupied volume ratio of the superabsorbent resin in the interspersed sections is higher than an occupied volume ratio of the superabsorbent resin in the areas surrounding the interspersed sections.

## Description

### Technical Field

The present invention relates to absorbent articles and methods for producing absorbent articles. Specifically, the present invention relates to an absorbent article including an absorbent body material having an accumulated absorbent fiber and a superabsorbent resin, and a method for producing that absorbent article.

### Background Art

Conventionally, an absorbent article including an absorbent body material having an accumulated absorbent fiber and a superabsorbent resin is known as an absorbent article for absorbing a predetermined fluid such as menstrual blood. Some absorbent articles are configured such that the superabsorbent resin is approximately uniformly present in a flat plane in the absorbent body material. When the absorbent article absorbs fluid, the superabsorbent resin swells when it holds the fluid, and particles of superabsorbent resin sometimes join together. Such joining of the superabsorbent resin particles may interfere with movement of the fluid absorbed in the absorbent article, resulting in reduction in the absorbing ability of the absorbent article.

In order to address the above problems, it is conceivable, for example, to intersperse in the absorbent body material, areas where superabsorbent resin is present, and densely gather the superabsorbent resin in those areas. If an absorbent body material having such a configuration is included, the fluid absorbed in the absorbent article moves in areas other than those areas in the absorbent body material where the superabsorbent resin is densely gathered, and consequently the fluid is easily dispersed within the absorbent body material. As a result, the absorbing ability of the absorbent article is maintained, even when the absorbent article has absorbed a large amount of fluid (see JP-A-9-504207).

### Disclosure of Invention

However, when absorbent fiber also is densely gathered in the areas where the superabsorbent resin is densely gathered, the absorbent fiber around the superabsorbent resin is pushed outward by the superabsorbent resin when the superabsorbent resin holds fluid and swells. As a result, bulging occurs in a part of the absorbent body material. That is, a part of the absorbent article bulges due to swelling of the superabsorbent resin, giving the wearer of the absorbent article a foreign-body sensation.

The present invention was made in view of these problems, and it is an advantage thereof to suppress bulging that occurs due to swelling of the superabsorbent resin within the absorbent article.

In order to address the above-described problems, an aspect of the invention is an absorbent article including an absorbent body material including an accumulated absorbent fiber and a superabsorbent resin, wherein the absorbent body material includes interspersed sections that are interspersed, and a densely gathered state of the absorbent fiber in the interspersed sections is less dense than a densely gathered state of the absorbent fiber in areas surrounding the interspersed sections, and an occupied volume ratio of the superabsorbent resin in the interspersed sections is higher than an occupied volume ratio of the superabsorbent resin in the areas surrounding the interspersed sections.

Features and advantages of the invention other than the above will become clear by reading the description of the present specification with reference to the accompanying drawings.

### Brief Description of Drawings

FIG. 1 is a schematic plan view that shows the configuration of an absorbent article 1;
FIG. 2 is a schematic plan view of an absorbent body material 12, and shows a skin face side of the absorbent body material 12;
FIG. 3 is a view that shows the cross-sectional structure of an interspersed section 13, and shows cross section A-A' in FIG. 2;
FIG. 4 shows a production flow of the absorbent article 1;
FIG. 5 is a flowchart of an absorbent body production step S100;
FIG. 6 schematically shows a process in which the absorbent body 10 is produced in the absorbent body production step S100;
FIG. 7 shows a mesh pattern 48 for forming a base material 15 of absorbent body material by accumulating pulverized pulp;
FIG. 8 is a diagram for illustrating a pattern roller 70 for supplying an SAP to the base material 15;
FIG. 9 shows a transition of the absorbent body material 12;
FIG. 10 schematically shows the manner of the absorbent body production step S100 according to a first modified example;
FIG. 11 shows the transition of an absorbent body material 12 according to the first modified example;
FIG. 12 shows a modified example of an indentation 70a of the pattern roller 70; and
FIG. 13 shows a configuration in which the thickness of an interspersed section 13 is smaller than that of the absorbent body material 12.

### List of Reference Numerals

1 ...absorbent article, 10 ... absorbent body, 10a ... swelling section, 10b... surrounding section, 11 ... thin paper (covering member), 12... absorbent body material, 13 ... interspersed section, 14... continuous section, 14a ... thick wall section, 14b... pulp accumulated layer, 15 ... base material, 15a... hole section, 20 ... surface sheet, 20a ... deep channel section, 25... side sheet, 30 ... back face sheet, 32 ... holding section, 40... suction drum, 42 ... pulp opening apparatus, 44... pulp scraping mechanism, 46 ... pulp supply roller, 48... mesh pattern (mold), 48a ... convex section, 48b... concave section, 50 ... thin paper supply roller, 60... suction conveyor (suction stage), 62... thin paper bending section, 70 ... pattern roller (roller), 70a... indentation, 71 ... SAP supply mechanism, 72 ... SAP feeder, 80... absorbent body cutter

### Best Mode for Carrying Out the Invention

At least the following matters will be made clear by reading the description of the present specification with reference to the accompanying drawings.

First, an absorbent article including an absorbent body material including an accumulated absorbent fiber and a superabsorbent resin, wherein the absorbent body material includes interspersed sections that are interspersed, and a densely gathered state of the absorbent fiber in the interspersed sections is less dense than a densely gathered state of the absorbent fiber in areas surrounding the interspersed sections, and an occupied volume ratio of the superabsorbent resin in the interspersed sections is higher than an occupied volume ratio of the superabsorbent resin in the areas surrounding the interspersed sections.

In such an absorbent article, areas where the superabsorbent resin is densely gathered (interspersed sections) are interspersed, and the fluid absorbed by the absorbent article moves in the areas surrounding the interspersed sections. That is, since the fluid can be easily dispersed within the absorbent body material, the absorbing ability of the absorbent article can be maintained. However, the fact that the superabsorbent resin is densely gathered in the interspersed sections (in other words, the occupied volume ratio of the superabsorbent resin is higher in the interspersed sections than in the areas surrounding the interspersed sections) means that in the interspersed sections, there is a higher possibility that bulging occurs in the absorbent article due to swelling of the superabsorbent resin.

On the other hand, since the densely gathered state of the absorbent fiber is less dense in the interspersed sections than in the areas surrounding the interspersed sections, in the interspersed sections, empty excess space is provided for swelling of the superabsorbent resin. With such empty excess space, it is possible to suppress the occurrence of bulging due to swelling of the superabsorbent resin. Specifically, although the possibility for the bulging to occur increases by densely gathering the superabsorbent resin in the interspersed sections, such possibility is cancelled by making the densely gathered state of the absorbent fiber in the interspersed sections less dense. As a result, it becomes possible to suppress the occurrence of bulging due to swelling of the superabsorbent resin in the absorbent article.

Also, the superabsorbent resin may be dispersed in the interspersed sections. In such a case, since more empty excess space is ensured around the superabsorbent resin, the effect of suppressing the occurrence of bulging is improved.

Also, a covering member for covering the absorbent body material may be included, and an embossing process for integrating the absorbent body material and the covering member may be performed only on positions that correspond to, of the interspersed sections and the areas surrounding the interspersed sections, the areas surrounding the interspersed sections. In such a case, it is possible to prevent the absorbent fiber and the superabsorbent resin in the interspersed sections from flowing outside of the interspersed sections. As a result, the densely gathered state of the absorbent fiber and the occupied volume ratio of the superabsorbent resin in the interspersed sections can be maintained in a state that suppresses the occurrence of bulging. Further, since the embossing process is not performed on the positions corresponding to the interspersed sections, the densely gathered state of the absorbent fiber in the interspersed sections does not change before and after the embossing process. Therefore, the absorbent fiber is not made high density in the interspersed sections, and the effect of suppressing the bulging is more effectively exhibited.

Also an absorbent article may have a longitudinal direction, a width direction, and a thickness direction, and an occupied volume ratio of the interspersed sections positioned in an end section along the longitudinal direction of the absorbent article relative to the end section is greater than an occupied volume ratio of the interspersed sections positioned in a center section along the longitudinal direction of the absorbent article relative to the center section. In such a case, the end sections will be arranged with more superabsorbent resin with great moisture-absorbing ability than in the center section. Thus, the moisture-absorbing ability, of the absorbent article, at the end sections side that contact the abdomen and buttocks of the wearer, is higher than that at the center section side. With such an absorbent article, in the vicinity of the abdomen and buttocks which tends to become hot and stuffy, more moisture can be absorbed. As a result, it is possible to suppress giving discomfort to the wearer due to becoming hot and stuffy.

Also, it is possible to realize a method for producing an absorbent article, the absorbent article including an absorbent body material that includes an accumulated absorbent fiber and a superabsorbent resin, the absorbent body material including interspersed sections, the method including: a first step of acquiring a base material of the absorbent body material including hole sections in positions corresponding to the interspersed sections, by accumulating the absorbent fiber in a mold, the mold including a base section and convex sections formed in positions corresponding to the interspersed sections in the base section; and a second step of supplying the absorbent fiber and the superabsorbent resin in the hole sections in order to acquire the absorbent body material from the base material, wherein in the second step, the absorbent fiber is supplied such that a densely gathered state of the absorbent fiber in the interspersed sections is less dense than a densely gathered state of the absorbent fiber in the areas surrounding the interspersed sections, and an occupied volume ratio of the superabsorbent resin in the interspersed sections is higher than an occupied volume ratio of the superabsorbent resin in areas surrounding the interspersed sections. With such a production method, the absorbent body material can be formed in a desired shape, and furthermore, the absorbent article that can suppress the occurrence of bulging can be provided.

Also, a configuration may be adopted in which in the second step, the absorbent fiber that has accumulated in adjacent portions in the base material adjacent to the hole sections is removed from the adjacent portions, and is supplied into the hole sections, such that the densely gathered state of the absorbent fiber in the interspersed sections is less dense than the densely gathered state of the absorbent fiber in the areas surrounding the interspersed sections, and the superabsorbent resin is supplied into the hole sections such that the occupied volume ratio of the superabsorbent resin in the interspersed sections is higher than the occupied volume ratio of the superabsorbent resin in the areas surrounding the interspersed sections. In such a case, when the absorbent fiber is supplied into the hole sections, it is not necessary to separately supply the absorbent fiber from the outside, and therefore the step of supplying the absorbent fiber and the superabsorbent resin into the hole sections can be simplified.

Also, a configuration may be adopted in which in the second step, a roller is used, the roller being configured rotatably and including indentations for containing the superabsorbent resin, and the base material is passed below the roller and while the surface of the base material is facing the outer circumference of the roller, the superabsorbent resin contained in the indentations is supplied from the indentations to the hole sections.

Also, the above method for producing an absorbent article may include the steps of: covering the absorbent body material with a covering member for covering the absorbent body material; and performing an embossing process for integrating the absorbent body material and the covering member only on positions where the interspersed sections in the absorbent body material are not present. In such a case, since the embossing process is not performed on the positions corresponding to the interspersed sections, the absorbent fiber is not made high density in the interspersed sections, so it is possible to provide the absorbent article capable of suppressing the occurrence of bulging more effectively.

### Absorbent Article According to Present Embodiment

### Overall Configuration of Absorbent Article

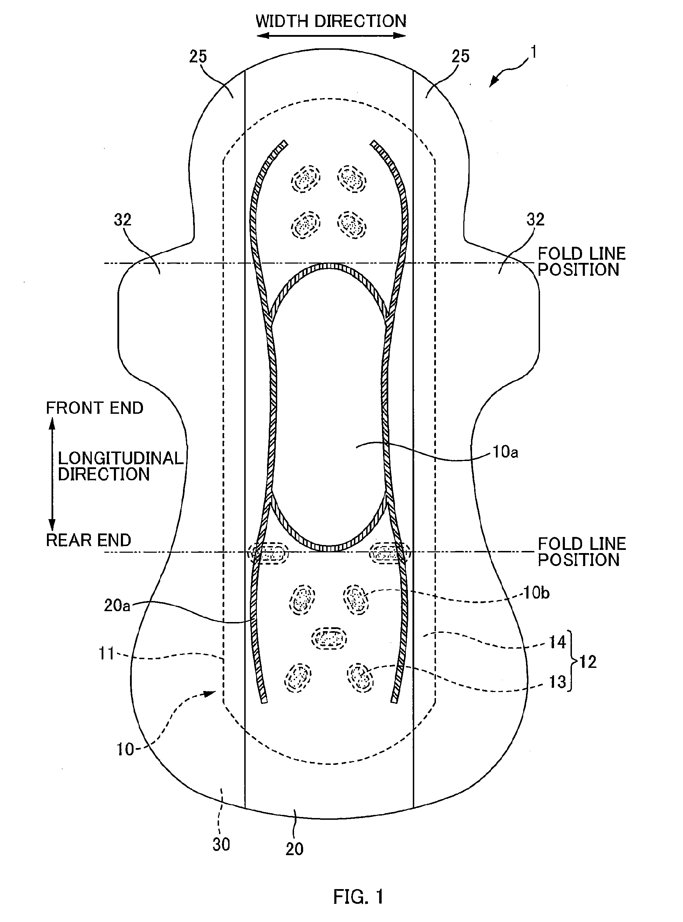

First, as an example absorbent article of the present embodiment, using a sanitary napkin as an example (referred to below as an absorbent article 1), a configuration example of the absorbent article 1 will be described using FIG. 1. FIG. 1 is a schematic plan view that shows the absorbent article 1. In FIG. 1, the longitudinal direction and the width direction of the absorbent article 1 are indicated with arrows. In the following description, among the surfaces of the absorbent article 1, the face that contacts the body of the wearer of the absorbent article lis referred to as a skin face, and the face that contacts an undergarment is referred to as an opposite face. Also, in the longitudinal direction of the absorbent article 1, the end that is positioned on the front side (abdominal side) of the wearer when worn is referred to as a front end, and the end that is positioned on the rear side (buttocks side) is referred to as a rear end. FIG. 1 shows the skin face side of the absorbent article 1.

The absorbent article 1 has an outer shape elongated in a predetermined direction, as shown in FIG. 1. The absorbent article 1 includes an absorbent body 10 having a substantially rectangular shape for absorbing fluid such as menstrual blood, a fluid-permeable surface sheet 20 that covers the surface of the skin face side of the absorbent body 10, a side sheet 25 disposed at both ends in the width direction of the absorbent body 10 on the skin face side of the absorbent body 10, and a back face sheet 30 provided on the opposite face side of the absorbent body 10.

This sort of absorbent article 1, in a portion from the front end section to the rear end section in the longitudinal direction of the absorbent article 1, is worn so as to contact a portion of the wearer's body from the buttocks to the abdomen. The absorbent article 1 can be folded up along the longitudinal direction, and when packaged as a product is in a state folded up at predetermined fold line positions (indicated by dashed lines in FIG. 1) (i.e., a state folded in three). Specifically described, fold line positions are present at two locations in the absorbent article 1 of the present embodiment. When folded at those fold line positions, the absorbent article becomes folded in three, and fold lines are formed at the fold line positions in the width direction of the absorbent article 1. Also, in the present embodiment, the fold lines are border lines that divide the front end section, the center section, and the rear end section in the longitudinal direction of the absorbent article 1. That is, in the longitudinal direction of the absorbent article 1, the portion toward the front end side from the fold line position of the front end side is referred to as a front end section, the portion toward the rear end side from the fold line position of the rear end side is referred to as a rear end section, and the portion between the two fold line positions is referred to as a center section. Thus, when the absorbent article 1 is worn, the center section contacts the wearer at the groin (around the menstrual blood discharge opening of the wearer), the front end section contacts the wearer from one end section at the groin to the abdomen, and the rear end section contacts the wearer from the other end section at the groin to the buttocks.

As shown in FIG. 1, the absorbent body 10 is attached in the center section in the width direction of the absorbent article 1, and the longitudinal direction of the absorbent body 10 and the longitudinal direction of the absorbent article 1 are aligned with each other. A swelling section 10a swelling toward the skin face side is formed in a portion that is positioned in the center in the longitudinal direction of the absorbent body 10, and positioned in the center in the width direction. The swelling section 10a is formed in an oval shape, and when the wearer is wearing the absorbent article 1, conforming to the shape of the groin of the wearer (that is, around the menstrual blood discharge opening of the wearer), the absorbent article 1 fits closely to the groin via the surface sheet 20. On the other hand, in the absorbent article 1, the end section in the longitudinal direction in an area where the absorbent body 10 is present contacts the abdomen or the buttocks of the wearer. That is, the length in the longitudinal direction of the absorbent body 10 is such that in the absorbent article 1, regions where both end sections in the longitudinal direction of the absorbent body 10 are present contact the abdomen and the buttocks of the wearer.

Also, the absorbent body 10 according to the present embodiment is configured from, for example, a thin paper 11 such as tissue paper and an absorbent body material 12. The absorbent body material 12 is configured from pulverized pulp (pulp in sheet form that has been pulverized into a fibrous state) as an example of "absorbent fiber", and granular superabsorbent polymer (below, abbreviated as SAP) as an example of "superabsorbent resin". In the absorbent body material 12, the pulverized pulp is accumulated in sheet-like form, and the SAP is mixed into the pulverized pulp in a densely gathered state (more precisely, in a space formed in the pulverized pulp). The absorbent body material 12 will be described in detail later.

The thin paper 11 is an example of a covering member, and is a sheet that covers the absorbent body material 12 in a surrounding manner. The thin paper 11 is fluid-permeable, and is a sheet with perforations that are smaller than the grains of SAP, and therefore has a function to prevent SAP from leaking outside of the thin paper 11. Furthermore, the thin paper 11 prevents the accumulated pulverized pulp from falling outside of the thin paper 11. Also, in order to integrate the thin paper 11 and the absorbent body material 12 covered by the thin paper 11, an embossing process is performed on a predetermined portion of the absorbent body 10 so that the predetermined portion is compressed to form embossing (below, also referred to as absorbent body embossing) in that portion.

The surface sheet 20 is a fluid-permeable sheet member, and is formed from woven or nonwoven cloth or a perforated plastic sheet or the like that has been formed from natural fiber such as pulp or cotton, cellulose fiber such as rayon, or a thermoplastic hydrophobic fiber such as polyethylene or polypropylene. The surface sheet 20 is included in the center section in the width direction of the absorbent article 1, has a width slightly wider than the absorbent body 10 in the width direction, and about the same length as the back face sheet 30 in the longitudinal direction, and covers the entire surface of the absorbent body 10.

The back face sheet 30 is a thermoplastic and fluid-impermeable sheet of polyethylene, polypropylene, or the like. The back face sheet 30 is formed sufficiently wider than the absorbent body 10, and the entire circumference of the outer edge section thereof is positioned to the outside of the outer edge section of the absorbent body 10. Also, on both sides in the width direction, holding sections 32 are formed protruding to the outside in the width direction. When the absorbent article 1 is worn, the holding sections 32 are, in a state folded back to the opposite face side, fixed to the undergarment. The back face sheet 30 according to the present embodiment is a thermoplastic and fluid-impermeable sheet of polyethylene, polypropylene, or the like, but it is also possible to use a sheet member in which thin paper, nonwoven cloth, or the like has been layered and that includes a thermoplastic and fluid-impermeable sheet.

The side sheet 25 is an appropriate nonwoven cloth such as air-through nonwoven cloth formed with a synthetic resin fiber or spun-bonded nonwoven cloth, or nonwoven cloth made up of spun-bonded/melted-blown/spun-bonded layers. The side sheet 25, in a state overlapping a portion of the surface sheet 20 (more precisely, both end sections in the width direction of the surface sheet 20), is included in the end sections in the width direction of the absorbent article 1.

In the absorbent article 1 configured in the above manner, the skin face of the absorbent body 10 and the surface sheet 20 are joined with hot-melt adhesive, and are more strongly joined by a deep channel section 20a formed by a deep channel embossing process of pressing in the thickness direction using a high-temperature pressing member. As shown in FIG. 1, the deep channel section 20a according to the present embodiment is configured from a portion that surrounds the swelling section 10a, and a portion extended from the front end section to the rear end section of the absorbent article 1 in the longitudinal direction on both sides of the swelling section 10a. Further, inside the deep channel section 20a, along the deep channel section 20a, a shallow bottom section and a deep bottom section whose channel depths differ from each other are alternately disposed. Due to this sort of deep channel section 20a being formed, when the center section in the longitudinal direction of the absorbent article 1 has been bent, both end sections of the absorbent article 1 in the longitudinal direction follow the center section and are also easily bent. That is, the deep channel section 20a is a bend-inducing section that facilitates three-dimensional bending in order to bring the portion that corresponds to the aforementioned swelling section 10a into closer contact along the body when the absorbent article 1 is worn. Further, the deep channel section 20a also has a function to, when menstrual blood or the like has flowed into the deep channel section 20a, suppress scattering of the menstrual blood or the like by facilitating penetration to a location that has been compressed with high density (i.e., a deep bottom section). Also note that in the present embodiment, the shallow bottom section and the deep bottom section are alternately disposed in the deep channel section 20a, but this is not a limitation; for example, the channel depth in the deep channel section 20a may be uniform.

Further, the back face sheet 30 is joined with hot-melt adhesive to each opposite face side of the absorbent body 10 and the surface sheet 20. Also, on the skin face side of the absorbent body 10, from a position slightly overlapping both side sections of the absorbent body 10 to on the back face sheet 30, the side sheet 25 is joined with hot-melt adhesive. At positions where the back face sheet 30, the absorbent body 10, the surface sheet 20, and the side sheet 25 overlap, an embossing process is performed with a pressing member heated to a low temperature, thus more strongly joining the absorbent body 10, the surface sheet 20, the side sheet 25, and the back face sheet 30. Furthermore, a round sealing process is performed in which the outer edge section of the absorbent article 1 is hot-melt bonded at a low temperature. As described above, the absorbent body 10, the surface sheet 20, the side sheet 25, and the back face sheet 30 are joined by an embossing process, hot-melt adhesive, or the like. As a result, the absorbent body 10 is sealed within a space formed by the surface sheet 20, the side sheet 25, and the back face sheet 30.

### Structure of Absorbent Body Material

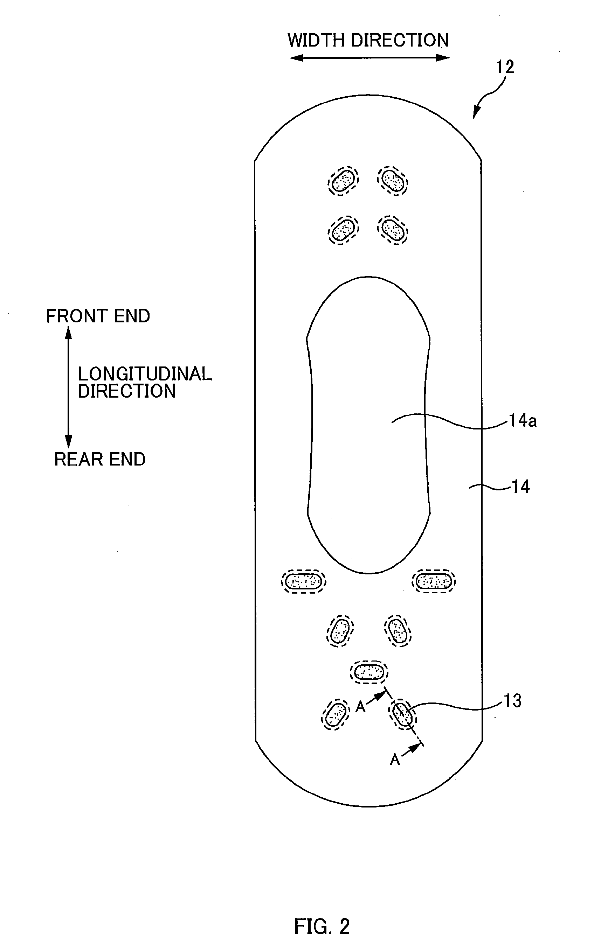

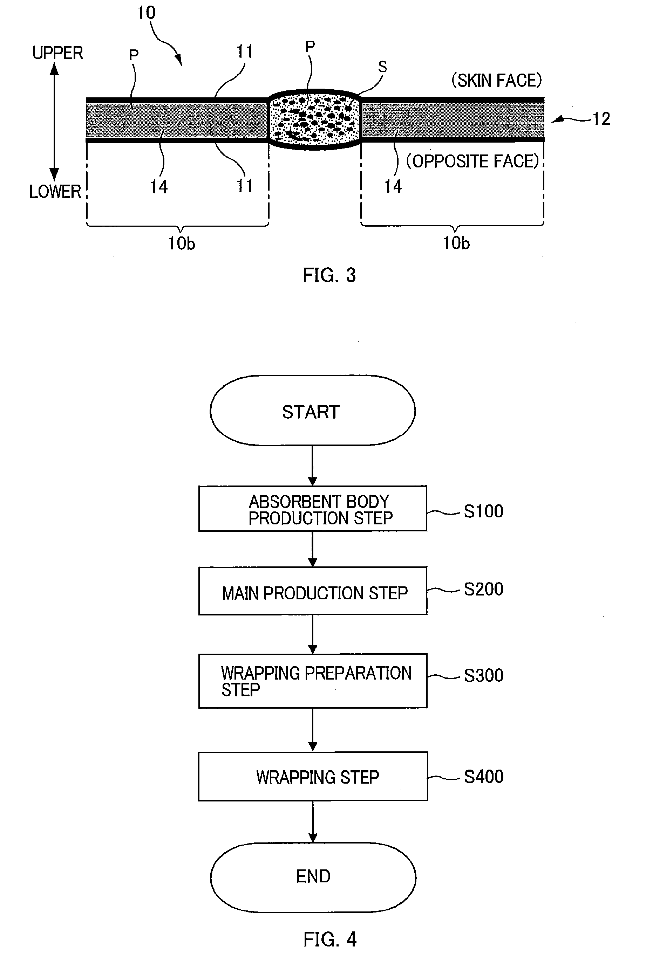

Next is a description of the structure of the absorbent body material 12 of the present embodiment, using aforementioned FIG. 1, and FIGS. 2 and 3. FIG. 2 is a schematic plan view of the absorbent body material 12, and shows the skin face side of the absorbent body material 12. FIG. 3 is a view that shows the cross-sectional structure of an interspersed section 13, and shows cross section A-A' in FIG. 2. In FIG. 2, the longitudinal direction and the width direction of the absorbent body material 12 are indicated with arrows.

The absorbent body material 12 is an approximately sheet-shaped member that is elongated in a predetermined direction as shown in FIG. 2, and as described above, is covered by the thin paper 11. In the portion positioned in the center in the longitudinal direction of the absorbent body material 12, and in the center in the width direction, which corresponds to the swelling section 10a of the absorbent body, a thick wall section 14a having a greater thickness than other sections is formed. Further, the aforementioned fold lines formed when the absorbent article 1 is folded up for wrapping are also borders that divide the center section and the end sections in the longitudinal direction of the absorbent body 10. In other words, the fold lines are borders that divide the center section and the end sections in the longitudinal direction of the absorbent body material 12. Accordingly, in the absorbent body material 12, positions that correspond to the fold line positions (indicated by the double-dotted chained line in FIG. 2) of the absorbent article 1 are positions that divide the center section and the end sections in the longitudinal direction of the absorbent body material 12. As described above, the absorbent body material 12 includes the pulverized pulp accumulated in sheet form (indicated by reference symbol P in FIG. 3, etc.) and SAP mixed into the pulverized pulp in an accumulated state (indicated by reference symbol S in FIG. 3, etc.). In the present embodiment, in the absorbent body material 12, areas are interspersed where SAP is densely gathered and the pulverized pulp in a densely gathered state is less dense than the areas surrounding those areas (hereinafter referred to as an "interspersed section 13").

More specifically described, the absorbent body material 12 of the present embodiment includes, as shown in FIG. 2, the interspersed sections 13 interspersed in the absorbent body material 12 and the continuous section 14 that is continuous around the interspersed sections 13. Each interspersed section 13 is, as shown in FIG. 2, an area in an oval shape in the plane defined by the longitudinal direction and the width direction of the absorbent body material 12. Each interspersed section 13 is, as shown in FIG. 3, formed from the skin face to the opposite face in the vertical direction of the absorbent body material 12 (i.e., the thickness direction of the absorbent body material 12). As shown in FIG. 1, in this embodiment, in the longitudinal direction of the absorbent body material 12, although a portion of the interspersed sections 13 is present straddling the center section and the rear end section, but approximately all of the interspersed sections 13 are present in the end sections in the longitudinal direction. In other words, the occupied volume ratio of the interspersed sections 13 positioned in the end sections in the longitudinal direction of the absorbent body material 12 relative to the end sections in the longitudinal direction is greater than the occupied volume ratio of the interspersed sections 13 positioned in the center section in the longitudinal direction relative to the center section in the longitudinal direction. Also, in the present embodiment, of the front end section and the rear end section, the occupied volume ratio of the interspersed sections 13 is greater for the rear end section.

The densely gathered state of pulverized pulp in the interspersed sections 13 is, as shown in FIG. 3, less dense than the densely gathered state thereof in the continuous section 14. Here, since the same pulverized pulp is densely gathered in the interspersed sections 13 and the continuous section 14, the densely gathered state of pulverized pulp means the weight of pulverized pulp included per unit volume (hereinafter referred to as the "pulp density"). Also, in the present embodiment, the pulp density is substantially equal with respect to the vertical direction of the body material 12 both in the interspersed sections 13 and the continuous section 14, and therefore the densely gathered state of pulverized pulp is expressed as the weight of the pulverized pulp included per unit area (hereinafter referred to as the "weight"). Accordingly, the weight of the pulverized pulp in the interspersed sections 13 is lower than the weight in the continuous section 14. The aforementioned absorbent body embossing is formed in the absorbent body 10, in the portion that corresponds to the areas surrounding the respective interspersed sections 13 (hereinafter referred to as the "surrounding section 10b"). In other words, an absorbent body embossing process is performed on the position corresponding to the continuous section 14, avoiding the position corresponding to the interspersed sections 13. For this reason, as shown in FIG. 3, the interspersed section 13 is thicker than the portion corresponding to the surrounding section 10b in the absorbent body material 12. The rigidity of the interspersed section 13 configured in this manner is smaller than that of the continuous section 14, and has better cushioning properties. Accordingly, when the absorbent article 1 is worn, the portion corresponding to the interspersed sections 13 gives a soft sensation to the skin of the wearer. Furthermore, as shown in FIG. 1, some of the interspersed sections 13 are present at the position corresponding to the fold line position used when folding the absorbent aritcle 1 (position indicated by the dashed line in FIG. 1). That is, in the present embodiment, interspersed sections 13 are present that overlap a fold line formed when the absorbent article 1 has been folded. Because interspersed sections 13 with less rigidity overlap the fold line in this manner, folding of the absorbent article 1 can easily be performed. Also, because the longitudinal direction of the interspersed sections 13 that overlap the fold line (i.e., the major axis direction of the oval interspersed sections 13) is aligned with the fold line, folding of the absorbent article 1 is still more easily performed.

On the other hand, because SAP is densely gathered in the interspersed sections 13, the occupied volume ratio of SAP in the interspersed sections 13 is higher than the occupied volume ratio around the interspersed sections 13 (i.e., the continuous section 14). Also, as described above, substantially all of the interspersed sections 13 are interspersed in the end sections in the longitudinal direction of the absorbent body material 12, and the occupied volume ratio of the interspersed sections 13 positioned in the end sections in the longitudinal direction of the absorbent body material 12 relative to those end sections in the longitudinal direction is higher than the occupied volume ratio of the interspersed sections 13 positioned in the center section in the longitudinal direction relative to that center section in the longitudinal direction. Note that in the absorbent body material 12 according to the present embodiment, SAP is mainly disposed in the interspersed sections 13, and the occupied volume ratio of SAP in each interspersed section 13 (i.e., the filling ratio of SAP in each interspersed section 13) is approximately fixed between the interspersed sections 13. Thus, in the end sections in the longitudinal direction, the occupied volume ratio of SAP is higher than in the center section in the longitudinal direction. That is, the proportion of space occupied by SAP relative to the volume of the end sections in the longitudinal direction of the absorbent body material 12 is higher than the proportion of space relative to the volume of the end sections in the longitudinal direction. From the above, more of the SAP, which has excellent moisture absorption, is disposed in the end sections in the longitudinal direction than in the center section in the longitudinal direction. Thus, in the absorbent article 1, the moisture-absorbing ability of the end sections in the longitudinal direction, which contact the abdomen and buttocks of the wearer, is greater than the moisture-absorbing ability of the center section in the longitudinal direction. With this sort of absorbent article 1, it is possible to absorb more moisture from the area around the abdomen and buttocks, which easily becomes hot and stuffy. As a result, it is possible to suppress giving discomfort to the wearer due to becoming hot and stuffy.

Further, as shown in FIG. 3, SAP is mixed with pulverized pulp in the interspersed section 13 with SAP dispersed therein. In short, in the interspersed section 13, a certain distance is ensured between particles of SAP, and SAP in a grain form are present in the space formed in pulverized pulp.

### Method for Producing Absorbent Article

Next is a description of the method for producing the absorbent article 1 of the present embodiment, using FIG. 4. FIG. 4 shows a production flow of the absorbent article 1.

The method for producing the absorbent article 1 includes an absorbent body production step S100 of producing the absorbent body 10; a main production step S200 of producing the absorbent article 1 using the absorbent body 10 produced in the absorbent body production step S100, the surface sheet 20, the side sheet 25, and the back face sheet 30; a wrapping preparation step S300 of preparing the absorbent article 1 for wrapping; and a wrapping step S400 of wrapping the absorbent article 1. In the present embodiment, the above steps are executed while materials and products of the absorbent article 1 are transported by a transporting apparatus such as a conveyor. Following is a detailed description of the absorbent body production step S100 in the production flow of the absorbent article 1.

### Absorbent Body Production Step

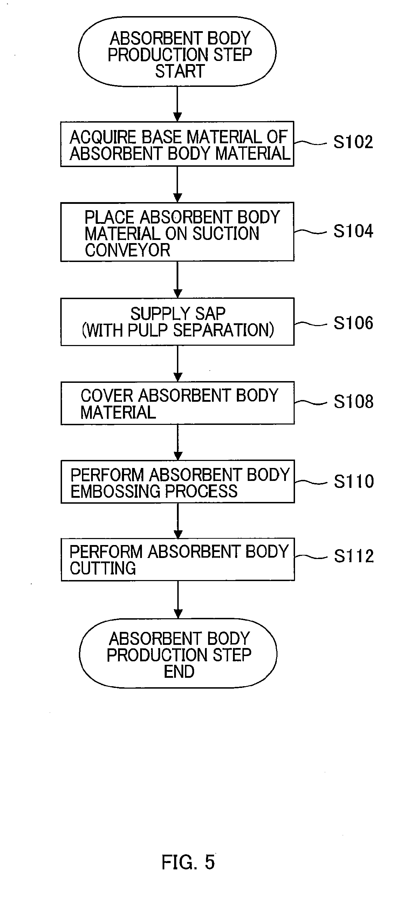

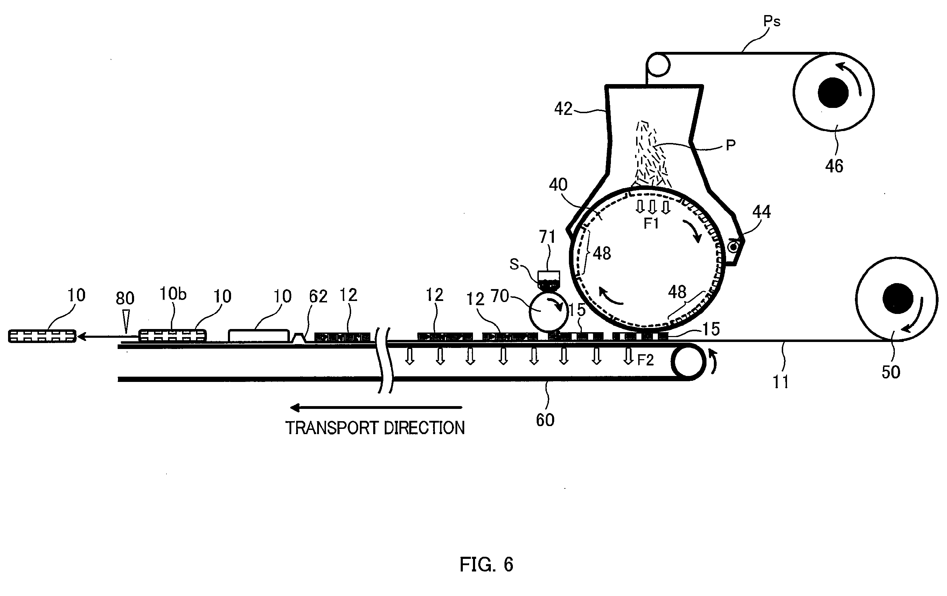

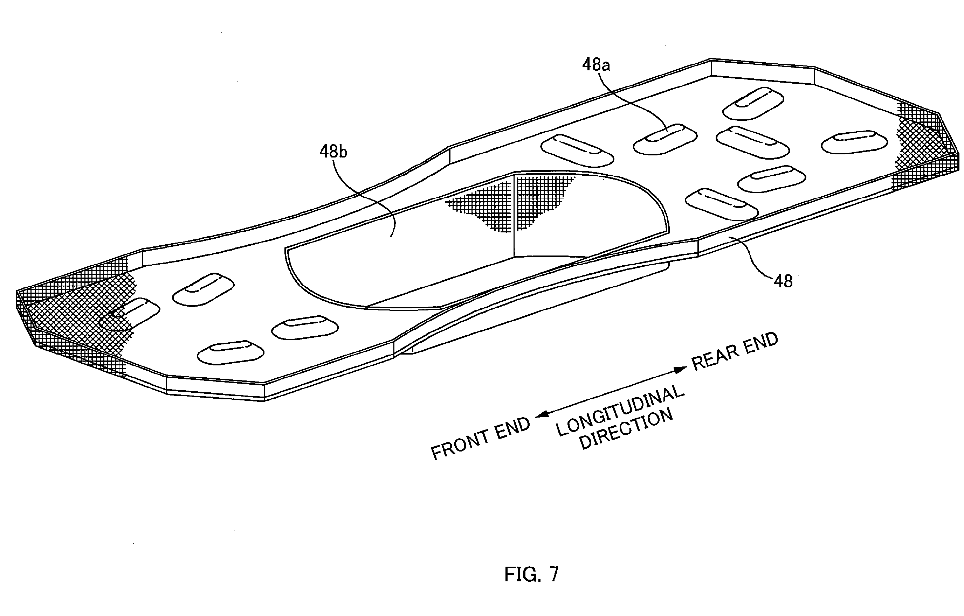

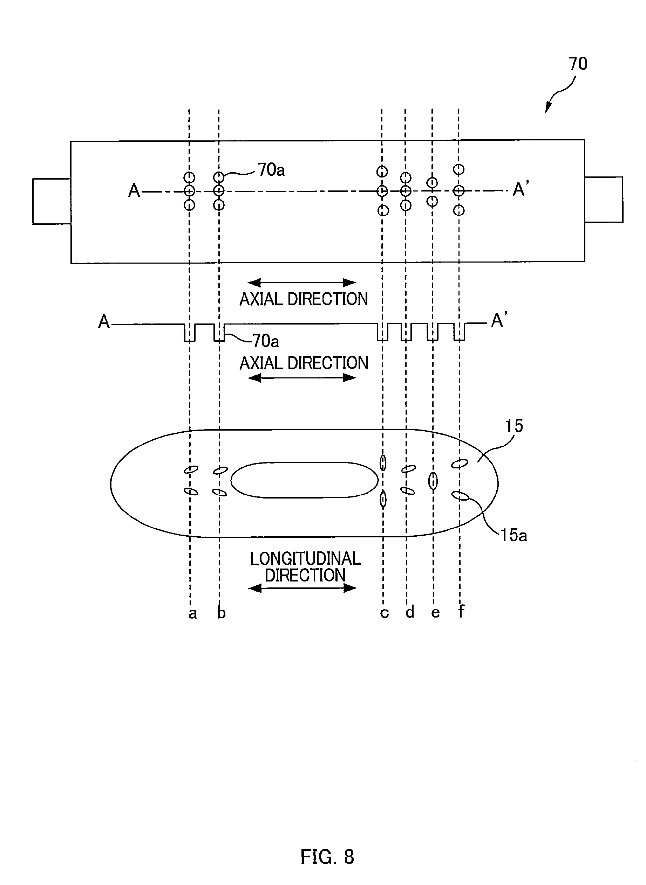

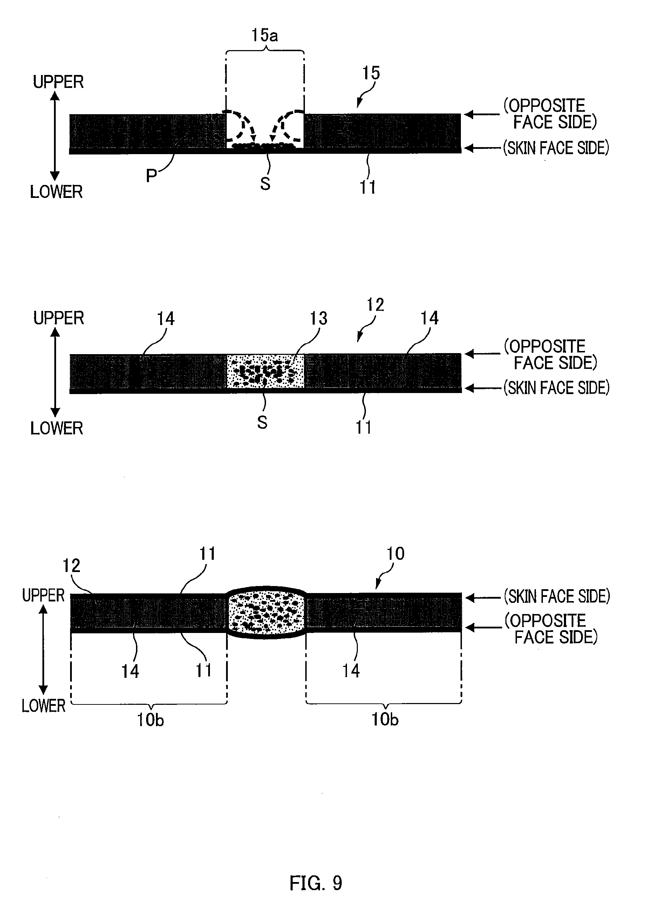

Following is a description of the absorbent body production step S100 using FIGS. 5 to 9. FIG. 5 is a flowchart of the absorbent body production step S100. FIG. 6 schematically shows a process in which the absorbent body 10 is produced in the absorbent body production step S100. FIG. 7 shows a mesh pattern 48 as an example of a "mold" for forming a base material 15 of absorbent body material by accumulating pulverized pulp. FIG. 8 is a diagram for illustrating a pattern roller 70 as an example of a "roller" for supplying SAP to the base material 15. FIG. 8 includes a front view of the pattern roller 70 (top diagram), an A-A' cross-sectional view of the pattern roller 70 (middle diagram), and a view showing the corresponding relationship between indentations 70a provided on the outer circumference of the pattern roller 70 and hole sections 15a provided in the base material 15 (bottom diagram). FIG. 9 shows a transition of the absorbent body material 12, and illustrates a state in which SAP is supplied to the base material 15 (top diagram), a state in which the interspersed section 13 is formed (middle diagram), and a state in which the absorbent body material 12 is attached in the absorbent article 1 as a final product in a state covered by the thin paper 11 (bottom diagram).

As shown in FIG. 5, the absorbent body production step S100 starts from Step S102 of acquiring the base material 15 of the absorbent body material. In the present embodiment, the base material 15 is acquired by accumulating pulverized pulp within the mesh pattern 48 shown in FIG. 7.

The mesh pattern 48 is a metal mold form having a mesh-like bottom face (in FIG. 7, for the sake of convenience of illustration, shown with only a part of the bottom face being mesh-like). When a suction apparatus (not shown) provided external to the mesh pattern 48 sucks air via the bottom face of the mesh pattern 48, due to the suction force of the suction apparatus, pulverized pulp that drops from above the mesh pattern 48 is sucked into the mesh pattern 48. Shortly thereafter, pulverized pulp is accumulated within the mesh pattern 48, and layered up to a state having a predetermined thickness. The mesh pattern 48 has a shape that corresponds to the shape of the aforementioned absorbent body material 12. That is, the mesh pattern 48 has an outer shape elongated in a predetermined direction, and approximately trapezoidal columnar convex sections 48a are interspersed in the bottom section (more precisely, both end sections in the longitudinal direction of the bottom section). Furthermore, a concave section 48b is provided in the center section in the longitudinal direction of the mesh pattern 48, and in the center section in the width direction, in order to form the thick wall section 14a of the absorbent body material 12.

By accumulating pulverized pulp within the mesh pattern 48 with the above sort of configuration, the base material 15 having the same outer shape as the absorbent body material 12 is acquired. That is, because the base material 15 is form-molded, by adjusting the mold form it is possible to acquire the base material 15 with a desired shape. Thus, the absorbent body material 12 also can be formed with a desired shape. The concave section 48b is provided in a portion in the center section in the longitudinal direction of the mesh pattern 48 according to this embodiment, and in the center section in the width direction, in order to form the thick wall section 14a of the absorbent body material 12, but this is not a limitation. For example, it is also possible to adopt a configuration in which the portion in the center section in the longitudinal direction of the mesh pattern 48, and in the center section in the width direction, is a flat face, and a base material 15 of an absorbent body material is formed that does not have the thick wall section 14a.

Also, the hole sections 15a are interspersed in the base material 15 (see the bottom diagram of FIG. 8). The hole sections 15a are formed at positions corresponding to the interspersed sections 13 of the absorbent body material 12. Such hole sections 15a are formed by the convex sections 48a that are provided in the bottom section of the mesh pattern 48. That is, when accumulating pulverized pulp within the mesh pattern 48, as a result of accumulating the pulverized pulp so as to avoid portions that correspond to the convex sections 48a (in other words, such that pulverized pulp does not enter the convex sections 48a), the hole sections 15a are formed at portions that correspond to the convex sections 48a. In the present embodiment, each side face of the convex sections 48a is mesh-like, but in order to prevent pulverized pulp from entering the hole sections 15a, each side face of the convex sections 48a may be sealed with tape, resin, or the like. In the case of a mesh pattern 48 having a flat bottom face, a configuration may be adopted in which convex sections 48a formed individually with rubber, resin, or the like are installed to the bottom face of the mesh pattern 48.

Step S102 of acquiring the base material 15 using this sort of mesh pattern 48 will be described in more detail with reference to FIG. 6. Mesh patterns 48 are disposed in a state such that on the outer circumference of a suction drum 40 into which the suction apparatus is built and that is rotatable in a predetermined rotation direction (the direction indicated by the arrows in FIG. 6), the longitudinal direction of the mesh patterns 48 is aligned along the direction of the rotational axis of the suction drum 40. Each mesh pattern 48 is disposed in a state with the opening facing the outside. While air around the suction drum 40 is being sucked into the suction drum 40 via the mesh patterns 48 by the suction apparatus (i.e., in the direction indicated by reference symbol F1 in FIG. 6), the mesh patterns 48 rotate as a single body with the suction drum 40. On the other hand, above the suction drum 40, a pulp opening apparatus 42 is provided in order to pulverize and open pulp in sheet form (indicated by reference symbol Ps in FIG. 6). When pulp Ps in sheet form is supplied to the pulp opening apparatus 42 from a pulp supply roller 46, the pulp in sheet form is pulverized and opened inside the pulp opening apparatus 42 to produce pulverized pulp. Then, as shown in FIG. 6, the produced pulverized pulp drops from above the suction drum 40 to the suction drum 40 side, and due to the suction force of the suction apparatus, is collected inside the mesh patterns 48 disposed on the outer circumference of the suction drum 40. When accumulation of pulverized pulp inside the mesh patterns 48 proceeds, with rotation of the suction drum 40, the mesh patterns 48 move to the position of a pulp scraping mechanism 44. Then, of the pulverized pulp that has been accumulated inside the mesh patterns 48, the pulp scraping mechanism 44 scrapes away excessively accumulated pulverized pulp (for example, pulverized pulp that has been accumulated on the convex sections 48a within the mesh patterns 48). By the above process, the base material 15 of absorbent body material is formed within the mesh patterns 48.

Afterward, due to further rotation of the suction drum 40, when the base material 15 and the mesh pattern 48 containing the base material 15 reach the lowest section of the suction drum 40, the base material 15 is separated from the mesh pattern 48, and delivered to the next process. When the base material 15 is separated from the mesh pattern 48, the hole sections 15a interspersed in the base material 15 are formed from one end in the thickness direction of the base material 15 (i.e., skin face) to the other end thereof (i.e., the opposite face). This is because the pulp scraping mechanism 44 scrapes away the pulverized pulp that has been accumulated on the convex sections 48a within the mesh patterns 48. Furthermore, as described above, since the convex section 48a in the mesh pattern 48 has an approximately trapezoidal columnar shape (that means, the side faces of the convex sections 48a are tapered faces), the base material 15 can be separated from the mesh pattern 48 without losing shape.

After being separated from the mesh pattern 48, the base material 15 is placed on a suction conveyor 60 serving as an example of a "suction stage" used in order to place the base material 15 while suction is performed (S104). Inside the suction conveyor 60 the unshown suction apparatus is included, and air is sucked into the suction conveyor 60 (i.e., in the direction indicated by reference symbol F2 in FIG. 6) by the suction apparatus. Then, in a state in which the base material 15 has been drawn to the side of the suction conveyor 60 by the suction force of the suction apparatus, the base material 15 is transported in a predetermined transport direction (indicated by the arrow in FIG. 6). On the placement face of the suction conveyor 60, in advance, the continuous strip-like thin paper 11 is supplied from a thin paper supply roller 50, and the base material 15 is placed on the thin paper 11. That is, the base material 15 is drawn to the side of the suction conveyor 60 via the thin paper 11, and transported along with the thin paper 11 in the transport direction. The base material 15 that has been formed within the mesh pattern 48 is sequentially placed on the suction conveyor 60, so as shown in FIG. 6, a plurality of units of the base material 15 are placed on the suction conveyor 60 with intervals therebetween. Note that in the present embodiment, the base material 15 is transported in a state in which, of the surfaces of the base material 15, the surface that comes to the skin face side when completed as the absorbent article 1 is facing the placement face of the suction conveyor 60. However, this is not a limitation; a configuration may also be adopted in which the base material 15 is transported in a state in which the face that comes to the skin face side is facing the opposite side from the suction conveyor 60 side (i.e., upward).

Next, pulverized pulp and SAP are supplied into the hole sections 15a in the base material 15 transported on the suction conveyor 60 (S106). The supply of pulverized pulp and SAP into the hole sections 15a is performed by the suction apparatus provided in the suction conveyor 60, and the pattern roller 70 that rotates in a predetermined rotation direction (the direction indicated by the arrow in FIG. 6) in order to supply SAP into the hole sections 15a. When the base material 15 passes below the pattern roller 70, SAP is supplied from the pattern roller 70 into the hole sections 15a in the base material 15 and pulverized pulp is supplied into the hole sections 15a by the suction force of the suction apparatus.

For a more specific description of the pattern roller 70, as shown in the top diagram in FIG. 8, the circular hole-like indentations 70a for containing SAP are interspersed on the outer circumference of the pattern roller 70. As shown in the middle diagram in FIG. 8, the indentations 70a have a fixed depth in the radial direction of the pattern roller 70. After SAP has been supplied from an SAP supply mechanism 71 provided above the pattern roller 70 into the indentations 70a, with rotation of the pattern roller 70, the indentations 70a containing SAP move to the side below the pattern roller 70. On the other hand, the base material 15 that has been placed on the suction conveyor 60 is transported towards the area below the pattern roller 70 in a state in which the longitudinal direction of the base material 15 is aligned along the axial direction of the pattern roller 70. Then, as shown in FIG. 6, the base material 15 passes below the pattern roller 70, and while the surface of the base material 15 is facing the outer circumference of the pattern roller 70 (more precisely, the portion of the outer circumference where the indentations 70a containing SAP are disposed), the SAP contained in the indentations 70a is sucked inside the hole sections 15a of the base material 15 by the suction force of the suction apparatus, and thus supplied into the hole sections 15a. Here, as shown in the top and bottom diagrams in FIG. 8, the interspersal pattern of the indentations 70a on the outer circumference of the pattern roller 70 corresponds to the interspersal pattern of the hole sections 15a in the base material 15. Thus, SAP contained in each indentation 70a is supplied into the hole section 15a at the position that corresponds to that indentation 70a. For example, in the top diagram in FIG. 8, SAP contained in the indentations 70a at the position indicated by letter a in the axial direction of the pattern roller 70 is supplied into the hole sections 15a at the position indicated by the letter a in the longitudinal direction of the base material 15. Also note that, in the top diagram in FIG. 8, of the three indentations 70a at the position indicated by letter a, SAP contained in the indentation 70a positioned in the middle is supplied to both of the two hole sections 15a in the position indicated by letter a.

In this manner, to the base material 15 in a state drawn to the suction conveyor 60, SAP is supplied to the hole sections 15a from the side of the face of the base material 15 in the position opposite to the face that faces the suction conveyor 60 (i.e., the face that comes to the opposite face side when completed as the absorbent article 1). At this time, suction resistance in the hole section 15a is quite smaller than the suction resistance in portions other than the hole sections 15a in the base material 15 (i.e., portions where pulverized pulp is accumulated), so SAP is intensively sucked into the hole sections 15a. As a result, SAP is supplied so that the occupied volume ratio of SAP is higher in the hole sections 15a than in the areas other than the hole sections 15a.

Moreover, in the present embodiment, the SAP contained in the indentations 70a in the pattern roller 70 is sucked inside the hole sections 15a by the suction force of the suction apparatus, and a process of removing part of the pulverized pulp that has been accumulated from adjacent sections adjacent to the hole sections 15a in the base material 15 is performed. Then, the removed pulverized pulp is supplied to the hole sections 15a so that the pulverized pulp and SAP are mixed together in the hole sections 15a.

Following is a description of the process of removing pulverized pulp with reference to FIG. 9.

When SAP contained in the indentations 70a in the pattern roller 70 is sucked into the hole sections 15a in the base material 15 by the suction force of the suction apparatus, as shown in the top diagram in FIG. 9, the pulverized pulp that has been accumulated in the top portion of the adjacent section (namely, portion on the opposite face side) is also sucked by the suction force of the suction apparatus. Accordingly, part of the pulverized pulp that has been accumulated in the top portion of the adjacent sections is removed toward the hole section 15a. In other words, the suction pressure of the suction apparatus is adjusted so as to remove the pulverized pulp and supply the removed pulverized pulp into the hole section 15a. Then, the removed pulverized pulp is mixed with SAP in the hole section 15a, and accumulates inside the hole section 15a. As a result, as shown in the middle diagram in FIG. 9, the absorbent body material 12 is formed from the base material 15. That is, the interspersed section 13 of the absorbent body material 12 is formed in the hole section 15a. Portions other than the hole sections 15a of the base material 15 become the continuous section 14 of the absorbent body material 12.

In addition, as described above, the suction pressure of the suction apparatus is adjusted so as to remove pulverized pulp from the adjacent sections, and furthermore, the amount of removed pulverized pulp is also adjusted by adjusting the suction pressure. The amount of removed pulverized pulp is adjusted such that when an interspersed section 13 is formed in the hole section 15a, the weight of pulverized pulp in the interspersed section 13 is lower than the weight in the continuous section 14. As a result of the removed pulverized pulp and SAP mixed in the hole section 15a, SAP is dispersed in pulverized pulp in an accumulated state in the interspersed section 13.

After the absorbent body material 12 has been formed with the base material 15, the thin paper 11 between the absorbent body material 12 and the suction conveyor 60 is bent so as to surround the absorbent body material 12, thus covering the absorbent body material 12 (S108). In the present embodiment, in order to bend the thin paper 11, a thin paper bending section 62 is provided on the surface of the suction conveyor 60, and the thin paper is bent when passing by the thin paper bending section 62, thus covering the absorbent body material 12. At this time, the end sections of the thin paper 11 in the width direction (the direction intersecting the transport direction) are overlapped and bonded on the upper face side of the absorbent body material 12, so the thin paper 11 becomes cylindrical.

Next, in order to integrate the thin paper 11 and the absorbent body material 12, a predetermined portion of the absorbent body material 12 in a state covered by the thin paper 11 is compressed, thus performing an absorbent body embossing process that forms absorbent body embossing at that predetermined portion (S110). In the present embodiment, the predetermined portion is, as described above, the surrounding section 10b that is the continuous section 14 and surrounds the interspersed sections 13. The absorbent body embossing process is performed by passing between two rollers that vertically oppose each other (not shown). For example, a protrusion of a predetermined shape is formed in the lower roller in an area where contact is made with a region where the absorbent body embossing is formed when the absorbent body material 12 has been transported (i.e., a region that corresponds to the surrounding section 10b), and the surface of the opposing upper roller is flatly formed. Due to the absorbent body material 12 surrounded by the thin paper 11 passing between those two rollers, the protrusion compresses both the thin paper 11 and the absorbent body material 12. The thin paper 11 and the absorbent body material 12 are compressed by the protrusion, and become a single body due to the formation of a plurality of absorbent body embossing. In the present embodiment, since the absorbent body embossing is formed in the region corresponding to the surrounding section 10b, as shown in the bottom diagram in FIG. 9, pulverized pulp that has been accumulated in the region corresponding to surrounding section 10b is compressed. For this reason, in the continuous section 14, pulverized pulp is made a higher density in the region correponding to the surrounding section 10b. It should be noted that the outer edge of the surrounding section 10b is oval-shaped as shown in FIG. 1, and the region between the outer edge of the interspersed section 13 and the outer edge of the surrounding section 10b forms the region corresponding to the surrounding section 10b. Absorbent body embossing is formed in the region corresponding to the surrounding section 10b, and the shape of the absorbent body embossing is determined depending on the shape of the protrusion provided in the lower roller, and the absorbent body embossing can be formed in any shape, including a grid shape, dot shape, or waveform. However, it is preferable to form the absorbent body embossing in a grid shape in order to form the absorbent body embossing while avoiding the regions that correspond to the interspersed sections 13.

Afterward, the integrated thin paper 11 and absorbent body material 12 (i.e., the absorbent body 10) are cut along the outer shape of the absorbent body material 12 by an absorbent body cutter 80 (S112). When each of the above steps (S102 to S112) is completed, the absorbent body production step S100 ends.

### Effectiveness of Absorbent Article of Present Embodiment

The absorbent article 1 of the present embodiment is an absorbent aricle including the absorbent body material 12 having accumulated pulverized pulp and SAP, in which the absorbent body material 12 includes the interspersed sections 13 that are interspersed, and the densely gathered state of pulverized pulp is less dense in the interspersed sections 13 than in the areas surrounding the interspersed sections 13, and the occupied volume ratio of SAP is higher in the interspersed sections 13 than in the areas surrounding the interspersed sections 13. Through this, it becomes possible to suppress the occurrence of bulging in the absorbent article 1 to prevent giving the wearer of the absorbent article 1 a foreign-body sensation.

That is, as described above, with a conventional absorbent article, SAP was approximately uniformly present in a flat plane in the absorbent body material 12. In particular, when SAP is present in a layer-like manner on the skin face side of the absorbent article, if the SAP holds fluid absorbed by the absorbent article and swells, particles of swollen SAP join together, thereby interfering with movement of the fluid to the opposite face side (i.e., the lower side of the absorbent article). As a result, the absorbed fluid accumulates on the skin face side of the absorbent body material 12, and it becomes difficult for fluid to be absorbed on the skin face side, so the problem occurs that absorbing ability of the absorbent article is reduced.

As an example of a measure for addressing such a problem, for example, it is conceivable to intersperse areas where SAP is present in the absorbent body material, and densely gather SAP in those areas, thus not allowing SAP to be approximately uniformly present on the skin face side of the absorbent body material. Thus, even when the absorbent article has absorbed a large amount of fluid, the absorbed fluid does not accumulate on the skin face side of the absorbent article, and is easily dispersed within the absorbent body material, so as a result, the absorbing ability of the absorbent article is maintained.

From the viewpoint of maintaining the absorbing ability of the absorbent article in this manner, it is important to adjust the arrangement of SAP in the absorbent body material. However, when only arrangement of SAP is adjusted without giving a consideration to the arrangement of pulverized pulp that constitutes the absorbent body material together with SAP, a new problem will occur in the absorbent article. That is, if pulverized pulp is also densely gathered in the area where SAP is densely gathered, pulverized pulp present around SAP is pushed out as a result of the SAP holding fluid and swelling. At this time, if pulverized pulp is densely gathered around SAP, a portion in the absorbent body material where SAP is densely gathered bulges locally. Accordingy, local bulging occurs in the absorbent article as well, which gives the wearer of the absorbent article a foreign-body sensation.

Also in the absorbent article 1 of the present embodiment, the interspersed sections 13 where SAP is densely gathered are interspersed in the absorbent body material 12, and therefore fluid absorbed by the absorbent article 1 is easily dispersed within the absorbent body material 12 (especially, within the continuous section 14). Therefore, even when the absorbent article 1 has absorbed a large amount of fluid, the absorbing ability of the absorbent article 1 is maintained. However, since SAP is densely gathered in the interspersed section 13 (in other words, the occupied volume ratio of SAP is higher in the interspersed sections 13 than in the areas surrounding the interspersed sections 13), in the interspersed sections 13, it is more probable that bulging occurs in the absorbent article 1 due to swelling of SAP.

On the other hand, the densely gathered state of pulverized pulp is less dense in the interspersed sections 13 than in the areas surrounding the interspersed sections 13. In other words, more space is present in the interspersed sections 13 than in the areas surrounding the interspersed sections 13, and therefore more empty excess space is provided for swelling of SAP. With such empty excess space, it is possible to suppress the occurrence of bulging due to swelling of SAP. Specifically, although the possibility for the bulging to occur increases by densely gathering SAP in the interspersed section 13, such possibility is cancelled by making the densely gathered state of pulverized pulp in the interspersed section 13 less dense. In this manner, local bulging in the absorbent body material 12 can be suppressed, which further enables suppression of the occurrence of bulging in the absorbent article. As a result, it becomes possible to prevent giving the wearer of the absorbent article 1 a foreign-body sensation.

Also, the occupied volume ratio of SAP is higher in the interspersed sections 13 than in the areas surrounding the interspersed sections 13, and the interspersed sections 13 have more empty excess space than the areas surrounding the interspersed sections 13, so the interspersed sections 13 are portions where moisture confined between the skin of the wearer and the absorbent article 1 due to perspiration or the like is actively absorbed. That is, because the interspersed sections 13 of the present embodiment are included, not only is bulging in the absorbent article 1 suppressed to prevent giving the wearer a foreign-body sensation, but also the absorbing ability of the absorbent article 1 is maintained even when a large amount of fluid has been absorbed by the absorbent article 1, and furthermore, it is possible to increase the moisture-absorbent properties of the absorbent article 1.

### Other Embodiments

Above, based on the above embodiments, an absorbent article according to the present invention and a method for producing the absorbent article were mainly described, but the above embodiments of the invention are for facilitating understanding of the invention, and are not for limiting the invention. The invention can of course be altered and improved without departing from the gist thereof and equivalents are intended to be embraced therein. In particular, embodiments of the invention are not limited by the numerical values stated in the above description, or the material qualities of each material. For example, in the above embodiments, pulverized pulp was described as an example of "absorbent fiber", but as other absorbent fibers, it is possible to use, for example, cellulose such as cotton, regenerated cellulose such as rayon or fibril rayon, semisynthetic cellulose such as acetate or triacetate, a fibrous polymer, or thermoplastic hydrophobic chemical fiber. Also, in the absorbent body material 12, other than pulverized pulp and SAP, granular deodorant, granular antibacterial material, granular coolant, or the like may also be densely gathered. Further, in the above embodiments, the thin paper 11 such as tissue was described as an example of a "covering member", but as other examples of covering members, it is possible to use a woven or nonwoven cloth formed from, for example, cellulose such as cotton, regenerated cellulose such as rayon or fibril rayon, semisynthetic cellulose such as acetate or triacetate, a fibrous polymer, or thermoplastic hydrophobic chemical fiber or the like.

Also, in the above embodiments, for the sake of convenience of description, the absorbent body 10 was described having a configuration including one absorbent body material 12 in the center in the width direction, but this is not a limitation. For example, a configuration may also be adopted in which side section absorbent bodies are respectively included along the longitudinal direction at both end sections of the absorbent body 10 in the width direction. Also, a configuration may be adopted in which, instead of side section absorbent bodies, solid gathers are included respectively at both of those end sections. Furthermore, in the above embodiments, the absorbent article 1 was folded in three. That is, a case was described in which fold line positions are present at two locations in the absorbent article 1, but this is not a limitation. For example, the absorbent article 1 can be folded in four.

Also, in the above embodiments, the interspersed sections 13 were interspersed in both end sections in the longitudinal direction of the absorbent body material 12, and not present in the center section, but this is not a limitation; an interspersed section 13 may be present in the center section in the longitudinal direction.

Also, in the above embodiments, a configuration was described in which SAP is densely gathered in the interspersed sections 13, and substantially no SAP is present in portions other than the interspersed sections 13 in the absorbent body material 12. That is, in the above emebodiments, a case was described in which SAP is concentratedly supplied in the hole sections 15a provided in the base material 15 of the present embodiment (below, also referred to as the original example). However, there is no limitation to this and SAP may be supplied also to portions other than the hole sections 15a in the base material 15. For example, an absorbent body material 12 may be formed in which SAP is supplied to the upper face of the base material 15 (i.e., the face that comes to the opposite side to the skin face of the absorbent body material 12), and the SAP is present in a layer on the upper face (hereinafter, an absorbent body material 12 according to the first modified example).

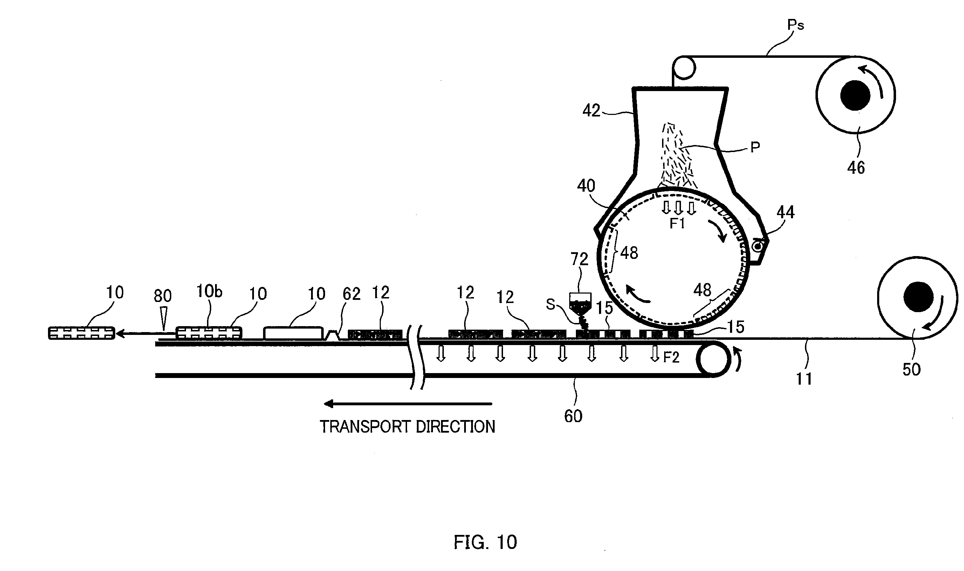

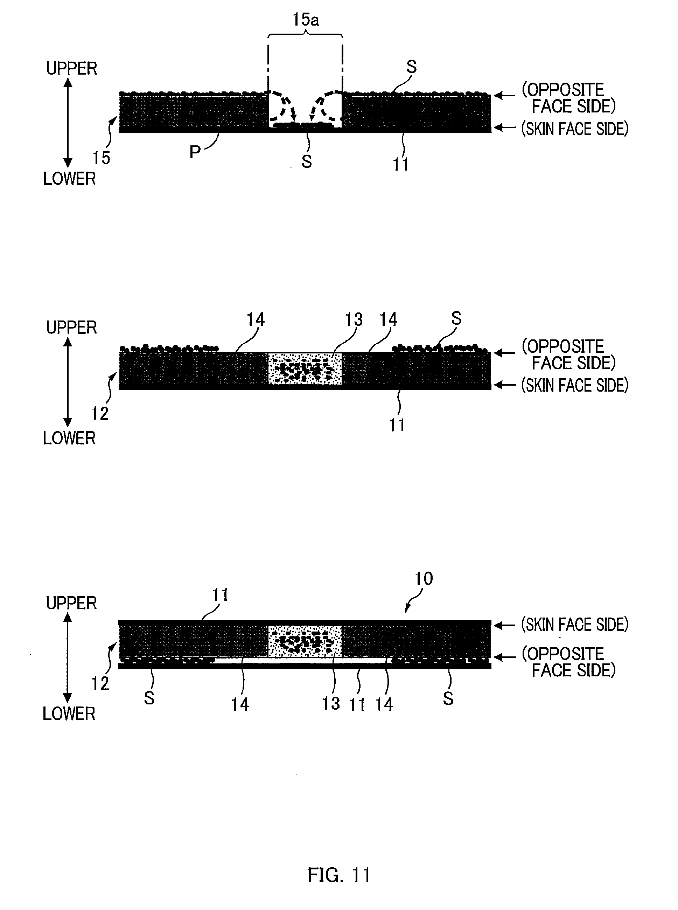

The absorbent body material 12 according to the first modified example will be specifically described with reference to FIGS. 10 and 11. FIG. 10 schematically shows the manner of the absorbent body production step S100 according to the first modified example, and corresponds to FIG. 6. FIG. 11 shows the transition of the absorbent body material 12 according to the first modified example, and shows the base material 15 with SAP supplied on the surface thereof (top diagram), the absorbent body material 12 formed from the base material 15 (middle diagram), and the absorbent body 10 formed by the absorbent body material 12 being covered by the thin paper 11 (bottom diagram).

As shown in FIG. 10, in the absorbent body production step S100 according to the first modified example, the pattern roller 70 is not used; an SAP feeder 72 is used as another mechanism that supplies SAP to the base material 15. This SAP feeder 72 has a conically shaped bottom section, and SAP is dropped from the bottom section. When the base material 15 transported in the transport direction by the suction conveyor 60 passes under the SAP feeder 72, SAP is dropped from the bottom section of the SAP feeder 72 to the face of the base material 15 on the side facing the SAP feeder 72. As a result, as shown in the top diagram in FIG. 11, SAP is distributed in a layer on the upper face of the base material 15. Note that in the first modified example as well, of the surfaces of the base material 15, the face that comes to the skin face side when completed as the absorbent article 1 is facing the placement face of the suction conveyor 60.

As in the original example, while SAP is supplied, the base material 15 is drawn to the suction conveyor 60 side by the suction apparatus provided in the suction conveyor 60. At this time, because the suction resistance in the hole sections 15a of the base material 15 is quite smaller than the suction resistance in portions other than the hole sections 15a in the base material 15, a larger amount of SAP is supplied into the hole sections 15a. Furthermore, in the first modified example as well, by the suction force by the suction apparatus, pulverized pulp is removed from the adjacent sections adjacent to the hole sections 15a, and that pulverized pulp is supplied to the hole sections 15a so as to be mixed with SAP in the hole sections 15a. It should be noted that as shown in the middle diagram in FIG. 11, when pulverized pulp is removed from the adjacent sections, part of SAP that has been accumulated on the upper face of the adjacent sections is also sucked into the hole sections 15a. By the above process, the absorbent body material 12 is formed from the base material 15, and SAP that has been accumulated remains on the opposite face side of the continuous section 14 in the absorbent body material 12. Then, when the absorbent body material 12 is installed in the absorbent article 1 in a state covered by the thin paper 11, as shown in the bottom diagram in FIG. 11, on the opposite face side, a SAP layer is formed between the the absorbent body material 12 (more precisely, the portion corresponding to the continuous section 14 in the absorbent body material 12) and the thin paper 11, and as a result, a larger amount of fluid can be held in the absorbent article 1. Above, an example was described in which SAP is supplied to the entire surface of the base material 15 by the SAP feeder 72, but from the viewpoint of appropriately supplying a predetermined amount of SAP to predetermined supply destinations, it is more desirable to intensively supply SAP into the hole sections 15a of the base material 15 using the pattern roller 70.

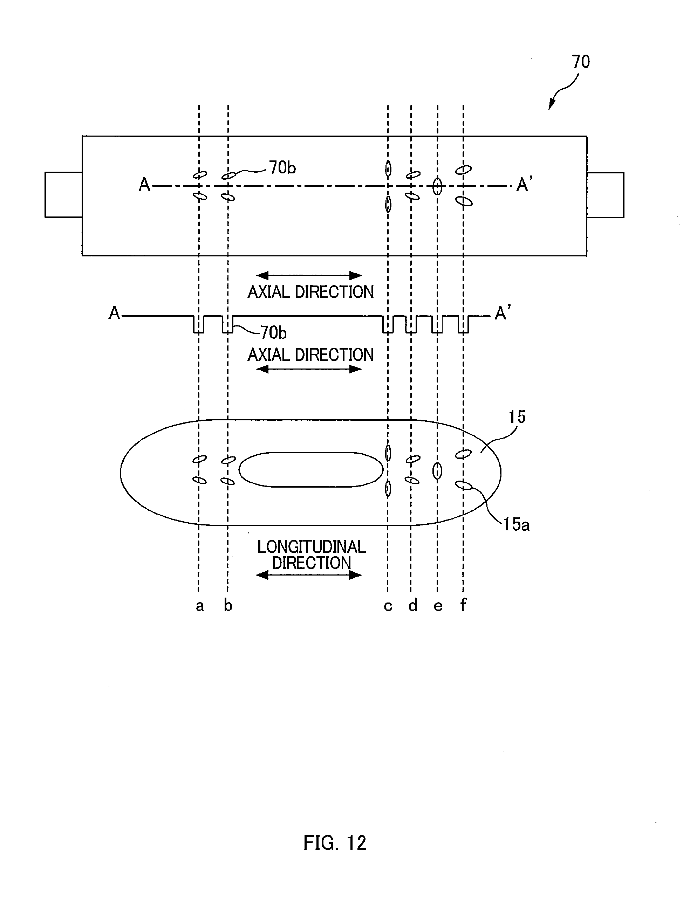

In the original example, the indentation 70a provided in the pattern roller 70 is a circular hole-shaped indentation, and the interspersal pattern of the indentations 70a corresponds to the interspersal pattern of the hole sections 15a in the base material 15. However, this is not a limitation; a configuration may also be adopted in which, for example, as shown in FIG. 12, the indentation 70a has an oval shape that matches the shape of the hole section 15a of the base material 15. FIG. 12 shows a modified example of indentation 70a of the pattern roller 70. When the indentation 70b has an oval shape, the amount of SAP supplied from each of the indentations 70b may be nonuniform. For example, in the oval indentation 70b, the amount of SAP supplied from an end section in the major axis direction and the amount of SAP supplied from the center section in the major axis direction may differ. On the other hand, in the case of circular indentation 70a, the amount of SAP supplied from each of the indentations 70a is uniform, so SAP is uniformly supplied to each hole section 15a of the base material 15. From the viewpoint of SAP being appropriately supplied into each hole section 15a in the above manner, the above embodiments are more desirable.

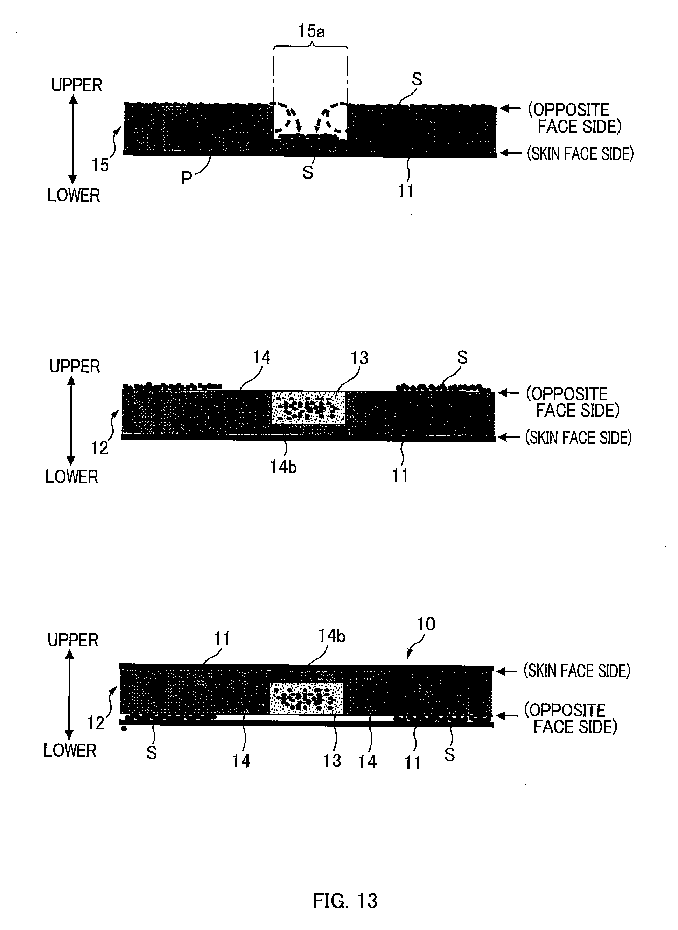

Also in the above embodiments, a configuration was adopted in which the interspersed sections 13 are formed in the vertical direction of the absorbent body material 12 (i. e. , the thickness direction of the absorbent body material 12) from the skin face to the opposite face. However, this is not a limitation. For example, as shown in FIG. 13, the thickness of the interspersed section 13 may be smaller than the thickness of the absorbent body material 12. FIG. 13 corresponds to FIG. 11, and shows a configuration in which the thickness of the interspersed section 13 is smaller than that of the absorbent body material 12. As shown in FIG. 13, below the interspersed section 13, a pulp accumulated layer 14b may be formed where pulverized pulp is densely gathered substantially in the same manner as in the continuous section 14. In order to obtain the absorbent body material 12 having such a configuration, a method may be adopted for example, in which when the base material 15 of the absorbent body material is acquired by accumulating pulverized pulp within the mesh pattern 48, pulverized pulp that has accumulated above the convex sections 48a within the mesh pattern 48 is left without scraping the pulverized pulp by the pulp scraping mechanism 44.

In the above embodiments, a configuration was adopted in which SAP is dispersed in the interspersed sections 13. However, this is not a limitation. For example, SAP may be present locally in the interspersed sections 13. However, if SAP is dispersed in the interspersed sections 13 as in the above embodiments, naturally more space is present around SAP. Accordingly, more empty excess space is insured for swelling of SAP, and the effect of suppressing the occurrence of bulging in the absorbent article 1 can be improved. With respect to this point, the above embodiments are more desirable.

Also in the above embodiments, a covering member that covers the absorbent bodymaterial 12 is included. However, this is not a limitation. For example, the absorbent body material 12 may not be covered. However, when the absorbent body material 12 is covered, it is possible to prevent pulverized pulp and SAP within the interspersed sections 13 from flowing out of the interspersed sections 13. As a result, the densely gathered state of pulverized pulp and the occupied volume ratio of SAP in the interspersed sections 13 are maintained in a state which can suppress the occurrence of bulging in the absorbent article 1. With respect to this point, the above embodiments are more desirable. It should be noted that woven or nonwoven cloth that has been formed from natural fiber or chemical fiber, for example, may be used for the covering member.

Also in the above embodiments, a configuration was adopted in which the absorbent body embossing process is performed for integrating the absorbent body material 12 and the thin paper 11 only on the positions, of the interspersed sections 13 and the areas surrounding the interspersed sections 13 (i.e., the positions corresponding to the surrounding sections 10b), that correspond to the areas surrounding the interspersed sections 13. However, this is not a limitation. For example, the absorbent body embossing process may be performed on the positions correponding to the interspersed sections 13. However, when the absorbent body embossing process is not performed on the positions corresponding to the interspersed sections 13, the densely gathered state of pulverized pulp in the interspersed sections 13 is maintained unchanged before and after the absorbent body embossing process. That is, pulverized pulp accumulated in the interspersed sections 13 is not made high density by the embossing process, and empty excess space for swelling of SAP is appropriately secured. Thus, an effect of suppressing the occurrence of bulging in the absorbent article 1 is more effectively exhibited. Also as described above, since the portion corresponding to the interspersed section 13 has a lower rigidity, when the absorbent body embossing process is performed on the position corresponding to the interspersed section 13, there is a risk that the thin paper 11 will be torn at a position corresponding to the interspersed section 13. For this reason, the absorbent body embossing process needs to be performed on the position avoiding the interspersed sections 13. With respect to this point, the above embodiments are more desirable.

Also in the above embodiments, a configuration was adopted in which in the step of supplying pulverized pulp and SAP in the hole sections 15a of the base material 15, part of the pulverized pulp that has been accumulated is removed from adjacent sections adjacent to the hole sections 15a of the base material 15, and that pulverized pulp is supplied into the hole sections 15a. However, this is not a limitation. For example, pulverized pulp may be supplied separately from a portion outside of the base material 15 to the hole sections 15a. However, in the above embodiments, a process of supplying pulp to be pulverized from the outside of the base material 15 to the hole sections 15a is omitted, so the step of supplying pulverized pulp and SAP into the hole sections 15a is simplified. With respect to this point, the above embodiments are more desirable.

Also, in the above embodiments, a configuration was adopted in which when supplying pulverized pulp and SAP into the hole sections 15a, in a state in which the base material 15 has been drawn to the side of the suction conveyor 60 by the suction force of a suction apparatus provided in the suction conveyor 60, pulverized pulp and SAP are supplied from the upper face side of the base material 15. However, this is not a limitation; a configuration may also be adopted in which the base material 15 is placed on a conveyor that is not provided with a suction apparatus.
However, when a suction apparatus applies suction force to the base material 15, because the suction resistance in the hole sections 15a of the base material 15 is quite smaller than the suction resistance in portions other than the hole sections 15a in the base material 15, pulverized pulp and SAP is easily sucked into the hole sections 15a. That is, in the above embodiments, it becomes possible to easily supply pulverized pulp and SAP into the hole sections 15a. With respect to this point, the above embodiments are more desirable.

Also, the shape, size, and the like of the convex sections 48a provided in the mesh pattern 48 may differ depending on the positions where they are disposed. For example, when an interspersed section 13 is provided in a portion of the absorbent body material 12 where the deep channel embossing process is performed (i.e., the portion where the deep channel section 20a is formed), it is more desirable that, among the convex sections 48a provided in the mesh pattern 48, the position of the uppermost face of the convex section 48a that forms that interspersed section 13 is lower than the position of the uppermost face of other convex sections 48a. In this case, it is possible to appropriately perform the deep channel embossing process even when an interspersed section 13 has been provided at a position where the deep channel embossing process is performed.

Specifically described, if the pulp density in an interspersed section 13 that has been provided at a position where the deep channel embossing process is performed is much less than the pulp density in the area surrounding that interspersed section 13 (specifically, the portion corresponding to the surrounding section 10b), when the deep channel embossing process has been performed, there is a risk that the surface sheet 20 will float up from the surface of the absorbent body material 12 and be torn. On the other hand, if, among the convex sections 48a provided in the mesh pattern 48, the position of the uppermost face of the convex section 48a that forms that interspersed section 13 provided at a position where the deep channel embossing process is performed is lower than the position of the uppermost face of other convex sections 48a, the pulp accumulated layer 14b where pulverized pulp is densely gathered is formed below that interspersed section 13 (see FIG. 13). Thus, because rigidity of the portion where that interspersed section 13 is positioned in the absorbent body material 12 is adjusted, it is possible to appropriately perform the deep channel embossing process even when an interspersed section 13 has been provided at a position where the deep channel embossing process is performed.

## Claims

1. An absorbent article comprising:
an absorbent body material including an accumulated absorbent fiber and a superabsorbent resin,
wherein the absorbent body material includes interspersed sections that are interspersed, and
a densely gathered state of the absorbent fiber in the interspersed sections is less dense than a densely gathered state of the absorbent fiber in areas surrounding the interspersed sections, and an occupied volume ratio of the superabsorbent resin in the interspersed sections is higher than an occupied volume ratio of the superabsorbent resin in the areas surrounding the interspersed sections.

2. An absorbent article according to claim 1, wherein
the superabsorbent resin is dispersed in the interspersed sections.

3. An absorbent article according to claim 1 or 2, wherein
a covering member for covering the absorbent body material is included, and
an embossing process for integrating the absorbent body material and the covering member is performed only on positions that correspond to, of the interspersed sections and the areas surrounding the interspersed sections, the areas surrounding the interspersed sections.

4. An absorbent article according to any of claims 1 to 3, wherein
the absorbent article has a longitudinal direction, a width direction, and a thickness direction, and
an occupied volume ratio of the interspersed sections positioned in an end section along the longitudinal direction of the absorbent article relative to the end section is greater than an occupied volume ratio of the interspersed sections positioned in a center section along the longitudinal direction of the absorbent article relative to the center section.

5. A method for producing an absorbent article, the absorbent article including an absorbent body material that has an accumulated absorbent fiber and a superabsorbent resin, the absorbent body material including interspersed sections, the method comprising:
a first step of acquiring a base material of the absorbent body material including hole sections in positions corresponding to the interspersed sections, by accumulating the absorbent fiber in a mold, the mold including a base section and convex sections formed in positions corresponding to the interspersed sections in the base section; and
a second step of supplying the absorbent fiber and the superabsorbent resin in the hole sections in order to acquire the absorbent body material from the base material,
wherein in the second step, the absorbent fiber is supplied such that a densely gathered state of the absorbent fiber in the interspersed sections is less dense than a densely gathered state of the absorbent fiber in areas surrounding the interspersed sections, and an occupied volume ratio of the superabsorbent resin in the interspersed sections is higher than an occupied volume ratio of the superabsorbent resin in the areas surrounding the interspersed sections.

6. A method for producing an absorbent article according to claim 5,
wherein
in the second step,
the absorbent fiber that has accumulated in adjacent portions in the base material adjacent to the hole sections is removed from the adjacent portions, and is supplied into the hole sections, such that the densely gathered state of the absorbent fiber in the interspersed sections is less dense than the densely gathered state of the absorbent fiber in the areas surrounding the interspersed sections, and
the superabsorbent resin is supplied into the hole sections such that the occupied volume ratio of the superabsorbent resin in the interspersed sections is higher than the occupied volume ratio of the superabsorbent resin in the areas surrounding the interspersed sections.

7. A method for producing an absorbent article according to claim 6,
wherein
in the second step,
a roller is used, the roller being configured rotatably and including indentations for containing the superabsorbent resin, and
the base material is passed below the roller and while the surface of the base material is facing the outer circumference of the roller, the superabsorbent resin contained in the indentations is supplied from the indentations to the hole sections.

8. A method for producing an absorbent article according to claim 6 or 7, comprising:
a step of covering the absorbent body material with a covering member for covering the absorbent body material; and
a step of performing an embossing process for integrating the absorbent body material and the covering member only on positions where the interspersed sections in the absorbent body material are not present.
